# EUROPÄISCHE PATENTANMELDUNG

(11) **EP 3 381 348 A1**
(43) Veröffentlichungstag der Anmeldung: **03.10.2018**
(21) Anmeldenummer: 18164602.7
(22) Anmeldetag: 28.03.2018
(51) Int. Cl.: A61B 1/00

(54) **MEDIZINISCHE VORRICHTUNG**

(30) Priorität: 28.03.2017 DE 102017106610
(71) Anmelder: Emmanouilidis, Nikos, 31515 Wunstorf (DE)
(72) Erfinder: Emmanouilidis, Nikos, 31515 Wunstorf (DE)
(74) Vertreter: Gramm, Lins & Partner Patent- und Rechtsanwälte PartGmbB

(57) **Zusammenfassung**

Die Erfindung betrifft eine medizinische Vorrichtung, z.B. für die Endoskopie und/oder Intubation, mit einem länglichen, in eine Körperöffnung eines Patienten einführbaren Hauptelement wie z.B. ein Katheter, ein Schlauch, ein Endoskop oder anderer länglicher flexibler Gegenstand, wobei die medizinische Vorrichtung ferner ein Referenzbauteil aufweist, das dazu eingerichtet ist, vor dem Einführen des Hauptelements in die Körperöffnung an einer definierten Stelle nahe der Körperöffnung am Patienten fixiert zu werden, wobei die medizinische Vorrichtung ferner eine Messvorrichtung aufweist, die über wenigstens ein Sensiermittel die Einführungstiefe des Hauptelements und/oder die axiale Orientierung des Hauptelements relativ zum Referenzbauteil erfasst und als Sensordaten zur weiteren Verarbeitung und/oder Speicherung bereitstellt.

## Beschreibung

Die Erfindung betrifft eine medizinische Vorrichtung, z.B. für die Endoskopie und/oder Intubation, mit einem länglichen, in eine Körperöffnung eines Patienten einführbaren Hauptelement wie z.B. ein Katheter, ein Schlauch, ein Endoskop oder ein anderer länglicher flexibler Gegenstand.

Für die chirurgische Planung einer Operation am Gastro-Intestinal Trakt ist eine genaue topographische Lokalisation der Erkrankung (z.B. eines Tumors) von entscheidender Bedeutung, um z.B. die Resektionsausmaße aber auch die Strategien einer operativen Rekonstruktion festzulegen.

Die topographische Bestimmung der erkrankten Region wird dabei meist entscheidend durch die endoskopische Diagnostik gewährleistet, wobei die erkrankte Region durch die Höhenangabe (in Zentimetern z.B. ab Zahnreihe, d.h. die Höhe zwischen Kamera und Zähnen, ab oral oder auch ab anal) aber auch durch Angaben der axialen Orientierung (rechts/links, vorne/hinten) genauer lokalisiert wird. Die Höhenangabe wird somit ab einer bestimmten Körperregion des Lebewesens (ab Zahnreihe, ab oral, ab anal) bis zur erkrankten Region gemessen. Für die Höhenangabe sind das in der Regel zwei Zahlen wie z.B. 20 cm bis 27 cm ab Zahnreihe, bzw. in axialer Orientierung meist eine ungenaue Sektoren Beschreibung wie z.B. halbe dorsale Zirkumferenz entsprechend 180° eines 360°-Systems.

Diese Lokalisation des pathologischen Befundes während der Endoskopie ist aber kein automatisierter Prozess, sondern erfolgt durch den Untersucher simultan durch zum Teil auch manuelles Ablesen der Zentimeter-Markierungen am Endoskop wie z.B. an der Zahnreihe (oder auch ab-anal), sowie oftmals auch unter manueller Unterstützung wie z.B. Spreizen der Rima ani oder auch Reposition des Kopfes des Patienten. Dabei muss der Untersucher den Blick vom Bildschirm abwenden, um die Markierungen direkt abzulesen, oder es ist ein Assistent notwendig, der das Ablesen synchron vornimmt. Dabei sind die Zahlenmarkierungen häufig so gelegen, dass das Endoskop wieder gedreht werden muss, um dieses ablesen zu können. Die Angaben in Zentimetern müssen dann entweder unmittelbar notiert werden oder der Untersucher merkt sich Orientierung, sowie Höhenangaben der pathologischen Befunde, um sie nach Abschluss der Untersuchung schriftlich zu dokumentieren.

Häufig wird bei der Untersuchung vergessen, synchrone Angaben zu Höhe bzw. axialer Orientierung zu geben bzw. es werden post-interventionem aus der Erinnerung falsche oder unvollständige Angaben zur Topographie, z.B. eines Tumors, gemacht. Im Falle mehrerer pathologischer Befunde, z.B. Polypen im Colon, multiplizieren sich die möglichen Fehler schnell. Selbst wenn eine Video-Aufzeichnung erfolgt ist, ist es später nicht möglich genaue Angaben in Zentimetern oder Grad zur Topographie des Befundes zu machen. Manchmal fällt dem Untersucher ein signifikanter pathologischer Befund auch erst bei zweiter Betrachtung wie z.B. eines synchron aufgenommenen Videos auf. Dann sind aber keine genaue Höhenangabe und keine Angabe zur axialen Orientierung möglich. Da es außerdem durch großzügige Skalierung am Endoskop wie auch durch interindividuelle Unterschiede der Untersucher beträchtlichen Interpretations-Spielraum beim Ablesen der Markierungen am Endoskop gibt, kann es leicht zu fehlenden, unvollständigen oder falschen topographische Angaben kommen.

Der Erfindung liegt daher die Aufgabe zugrunde, eine verbesserte medizinische Vorrichtung der Eingangs genannten Art zu schaffen.

Die Aufgabe wird mit einer medizinischen Vorrichtung mit den Merkmalen des Anspruchs 1 gelöst. Vorteilhafte Ausgestaltungen sind in den Unteransprüchen beschrieben.

Es wird vorgeschlagen, dass die medizinische Vorrichtung ein Referenzbauteil aufweist, das dazu eingerichtet, vor dem Einführen des Hauptelementes in die Körperöffnung an einer definierten Stelle nahe der Körperöffnung am Patienten fixiert zu werden, wobei die medizinische Vorrichtung ferner eine Messvorrichtung aufweist, die über wenigstens ein Sensiermittel die Einführungstiefe des Hauptelementes und/oder die axiale Orientierung des Hauptelementes relativ zum Referenzbauteil erfasst und als Sensordaten zur weiteren Verarbeitung und/oder Speicherung bereitstellt.

Eine derartige medizinische Vorrichtung hat den Vorteil, eine exakte und standardisierte Messung und Dokumentation während der Anwendung der medizinischen Vorrichtung ermöglicht wird. Auf diese Weise kann die topographische Bestimmung einer erkrankten Region durch endoskopische Diagnostik genauer bestimmt werden als bei den herkömmlichen medizinischen Vorrichtungen. Die erfindungsgemäße medizinische Vorrichtung erhöht damit die Zuverlässigkeit der Diagnostik im Bereich der Endoskopie. Zudem hat es den Vorteil, dass der Anwender nicht mehr auf manuelles Ablesen der Einführungstiefe und der axialen Orientierung des länglichen Hauptelementes angewiesen ist. Somit kann der Anwender die Konzentration auf die Untersuchung richten, was die Fehlerquote der Diagnostik verringert und eine lückenlose Dokumentation der Untersuchung ermöglicht.

Die medizinische Vorrichtung kann ein Bildanzeigegerät aufweisen oder damit verbunden sein, wobei die Vorrichtung dazu eingerichtet ist, in auf dem Bildanzeigegerät dargestellten Bilddaten in Echtzeit die von der Messvorrichtung bereitgestellten Sensordaten, die die Einführungstiefe und/oder die axiale Orientierung wiedergeben, einzublenden. Auf diese Weise ist für den Anwender jederzeit die Lage der medizinischen Vorrichtung in dem Patienten ersichtlich. Ferner vorteilhaft ist es, wenn die medizinische Vorrichtung dazu eingerichtet ist, die Sensordaten mit einem Zeitbezug zu speichern, insbesondere mit einem Zeitbezug zu den auf dem Bildanzeigegerät dargestellten Bilddaten.

Bilddaten sind Dateien, in der ein digitales Bild gespeichert ist, wobei diese Dateien von einer Recheneinheit, beispielsweise einem Computer, wieder in analoge Daten zurück gewandelt und auf einem Bildanzeigegerät visualisiert werden können. Derartige Bilddaten können zum Beispiel von einer Bilderfassungseinheit am distalen Ende des länglichen Hauptelement wie bei einem Endoskop aufgenommen, an die Messvorrichtung zur Weiterverarbeitung übertragen und auf der Recheneinheit durch gespeichert werden. Vorteilhafterweise ist die Bilderfassungseinheit eine Kamera, so dass eine exakte Reproduktion des Untersuchungsvorganges zu einem späteren Zeitpunkt möglich ist. Die Bilddateien können als Rastergrafik, die aus Pixeln zusammengesetzt ist, gespeichert werden. Die Dateien können zum Beispiel in einem Video-Format wie zum Beispiel MPEG und/oder in einem Bild-Format wie zum Beispiel JPEG gespeichert werden.

Auf diese Weise kann die Dokumentation der topografischen Bestimmung zu einem späteren Zeitpunkt beliebig oft abgespielt, befundet oder auch dem Chirurgen demonstriert werden, indem die Daten auf einem Datenträger gespeichert werden.

Das längliche Hauptelement der medizinischen Vorrichtung kann durch wenigstens eine Ausnehmung des Referenzbauteils führbar sein. Das Referenzbauteil kann dabei als hohles Element ähnlich eines Rohrstückes ausgebildet sein. Ferner vorteilhaft ist es, wenn eine Null-Kalibrierung zur Bestimmung der Ausgangsposition des länglichen Hauptelementes nach Einführung des länglichen Hauptelementes in das Referenzbauteil erfolgt. Eine Null-Kalibrierung kann dabei zum Beispiel automatisch durch Einführen des länglichen Hauptelementes in das Referenzbauteil oder manuell durch den Anwender erfolgen. Auf diese Weise kann ein mehrteiliges System bereitgestellt werden, das vor der Einführung des länglichen Hauptelementes in den Patienten eine Null-Kalibrierung ermöglicht und damit eine exakte Bestimmung der Einführungstiefe und/oder der axialen Orientierung des Hauptelementes relativ zum Referenzbauteil ermöglicht.

Das Referenzbauteil kann ein Mundstück haben, wobei das Mundstück wenigstens einen Beißring hat und dazu eingerichtet ist, das Referenzbauteil zu fixieren. Dies kann z.B. derartig erfolgen, dass das Referenzbauteil im Bereich des Mundes des Patienten angeordnet wird und der Patient das Referenzbauteil in seiner Position durch Zubeißen auf den Beißring fixiert hält.

Das Referenzbauteil kann zur verbesserten Reinigung mehrteilig aufgebaut sein. Auf diese Weise ist es möglich, die einzelnen Komponenten des Referenzbauteils auseinanderzubauen und separat zu desinfizieren. Zudem wird ein einfacher Austausch der einzelnen Komponenten ermöglicht.

Das Sensiermittel kann ein Laser-Sensor und/oder Kugel-Sensor und/oder eine Hall-sonde und/oder ein Induktions-Sensor und/oder ein Licht-Sensor und/oder ein Optischer-Sensor und/oder ein Wälzrollen-Sensor sein. Auf diese Weise wird ein Sensiermittel bereitgestellt, das auf einfache Weise in das Referenzbauteil integriert und eine Bestimmung der Einführungstiefe und/oder der axialen Orientierung des Hauptelementes erfolgen kann.

Bei einem Licht-Sensor, insbesondere einem Laser-Sensor, wird Licht unter Ausnutzung des photoelektrischen Effekts in ein digitales Signal umgewandelt. Dabei wird eine lichtempfindliche Fläche durch einfallende Strahlung angeregt wodurch ein Elektron aus dem Festkörper gelöst wird, indem ein Photon absorbiert wird. Die auftretende Spannung kann gemessen und somit ein digitales Signal erzeugt werden, dass auf einem Bildanzeigegerät wieder ausgegeben werden kann. Für eine Licht-Sensor Auslegung könnte das längliche Hauptelement reflektierend ausgebildet sein, wobei ein Licht-Sensor reflektierendes Licht einer Lichtquelle wie zum Beispiel eines Lasers erfassen kann.

Bei einem Kugel-Sensor wird durch wenigstens zwei Kugeln jeweils eine Welle bewegt, wobei die Wellen jeweils eine Scheibe mit Schlitzen am Ende der Welle in Rotation versetzen. Eine Lichteinheit und eine Fotozelle sind vor bzw. hinter den Schlitzen angeordnet. Durch die Wellen wird an den Schlitzen ein Takt aufgebaut, der grafisch umgesetzt werden kann und dadurch die Einführungstiefe und die axiale Orientierung des länglichen Hauptelementes gespeichert und dargestellt werden kann. Der Wälzrollen-Sensor funktioniert nach dem gleichen Prinzip wie der Kugel-Sensor, es können bei diesem Sensor aber anstatt der Kugeln jede Form von eines rollenden Körpers wie zum Beispiel einer Zylinderrolle verwendet werden.

Bei einem Hall-Sensor wird der Hall-Effekt zur Messung von Magnetfeldern verwendet. Dabei wird der Hall-Sensor von einem Strom durchflossen und in ein Magnetfeld eingebracht. Dieser Vorgang erzeugt eine Spannung, die proportional zur Flussdichte und Strom ist. Durch diese Spannung kann auf die Einführungstiefe des länglichen Hauptelementes geschlossen werden. Dabei kann das längliche Hauptelement mit magnetischen Bestandteilen in Form von Armierungen versehen sein, um beim Verschieben eine Spannung am Hall-Sensor zu erzeugen.

Bei einem Induktions-Sensor wird die Änderung der Induktivität, bzw. die Magnetfeldänderung, durch Lageänderung relativ zu einem leitfähigen oder ferromagnetischen Teil gemessen. Dabei wird durch eine Spule ein Magnetfeld erzeugt, wobei durch Verschieben des längliche Hauptelement innerhalb des Magnetfeldes eine Magnetfeldänderung erreicht wird, die gemessen und als digitales Signal gespeichert oder weiterverarbeitet werden kann. Dabei muss das längliche Hauptelement über seine Länge leitfähige oder ferromagnetische Bestandteile haben, damit eine Änderung des Magnetfeldes erreicht werden kann. Die leitfähigen Bestandteile können zum Beispiel als ferromagnetische Armierungen innerhalb des länglichen Hauptelementes ausgebildet sein.

Die Erfindung wird nachfolgend anhand von Ausführungsbeispielen unter Verwendung von Zeichnungen näher erläutert. Es zeigen:
- Figur 1: - Medizinische Vorrichtung in einer schematischen Darstellung;
- Figur 2: - Anzeigegerät während der Messung einer Endoskopie;
- Figur 3: - Beispielsequenzen einer Messung während einer Endoskopie;
- Figur 4: - Konzeptionellen Aufbau eines Mundstückes mit einem Referenzbauteil und einem Laser-Sensor;
- Figur 5: - Konzeptionellen Aufbau eines Mundstückes mit einem Referenzbauteil und einem Kugel-Sensor;
- Figur 6: - Konzeptionellen Aufbau eines Mundstückes mit einem Referenzbauteil und einem Induktions-Sensor;
- Figur 7: - Konzeptionellen Aufbau eines Mundstückes mit einem Referenzbauteil und einem Hall-Sensor.

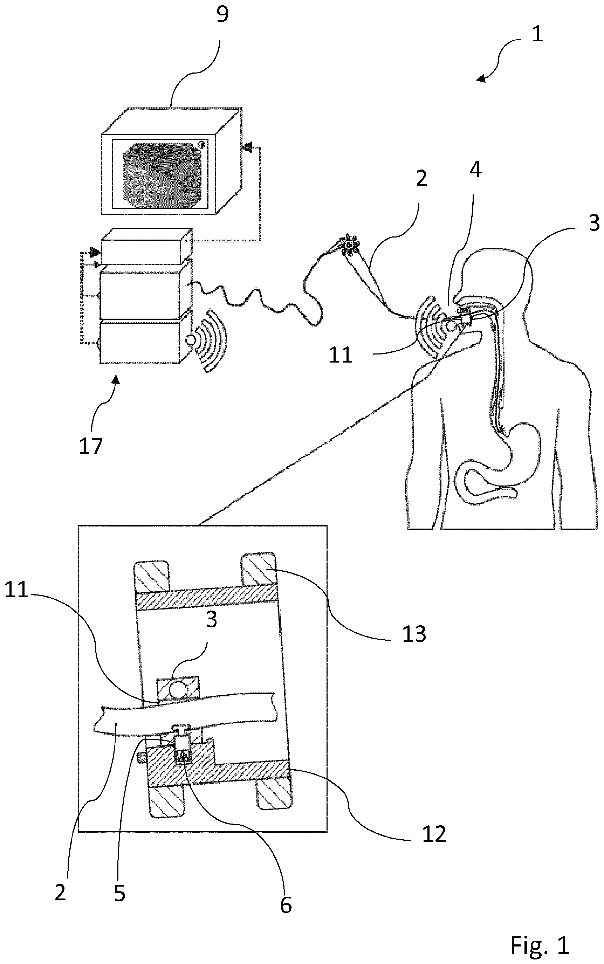

Figur 1 zeigt eine schematische Darstellung einer medizinischen Vorrichtung 1 mit einem länglichen Hauptelement 2 und einem Referenzbauteil 3, wobei das Referenzbauteil 3 in einer Körperöffnung 4 eines Patienten angeordnet ist. Die medizinische Vorrichtung 1 hat weiterhin eine Messvorrichtung 5, die mittels Sensiermittel 6 die Einführungstiefe und die axiale Orientierung des länglichen Hauptelementes 2 ermittelt und auf einem Bildanzeigegerät 9 wiedergibt. Die Sensiermittel 6 können dabei als Laser-Sensor 6a, Kugel-Sensor 6b, Induktions-Sensor 6c und/oder Hall-Sensor 6d ausgebildet sein, wobei die Sensiermittel 6 an dem Referenzbauteil 3 angeordnet sein können.

In der Vergrößerung wird deutlich, dass das Referenzbauteil 3 innerhalb eines Mundstückes 12 angeordnet ist, wobei das Mundstück 12 einen Beißring 13 zur Fixierung in der Körperöffnung 4 des Patienten hat. Das Referenzbauteil 3 hat eine Ausnehmung 11, wobei das längliche Hauptelement 2 durch die Ausnehmung 11 hindurchgeführt ist. Es ist von Vorteil, wenn nach Einführung des Hauptelementes 3 in die Ausnehmung 11 des Referenzbauteils 3 eine Null-Kalibrierung zur exakten Lagebestimmung des Hauptelementes 2 erfolgt.

Nachfolgend wird das Referenzbauteil 3 auch als DOME-Modul 3 (Distance and Orientation Module for Endoscopy) und das Hauptelement 2 als Endoskop 2 bezeichnet. Die Anwendung ist aber nicht auf ein Endoskop 2 beschränkt.

Die technische Lösung ermöglicht eine real-time Messung der Einführungstiefe 7 und axialen Orientierung 8 des Endoskops 2, sowie einer synchronen VIDEO-in-VIDEO Projektion der Messdaten in das Video der laufenden endoskopischen Untersuchung. Dieses Video mit VIDEO-in-VIDEO Messdaten wird dann aufgenommen und auf einem Computer 17 archiviert. Dieses Video kann dann frei nach Belieben und jederzeit am Bildanzeigegerät 9 oder einem Computer befundet, demonstriert oder auch zu tele-medizinischen Zwecken kopiert und verschickt werden.

Bei der Untersuchung wird das Endoskop 2 in ein speziell modifiziertes oder angefertigtes Mundstück 12 (oder Anal-Einsatz) eingeführt und bei Intubationsbeginn eine Null-Kalibrierung von Einführungstiefe 7 und axialer Orientierung 8 vorgenommen.

Das Mundstück 12 beinhaltet dabei eine Messvorrichtung 5 mit Sensiermittel 6, welche in spezieller Ausfertigung die Bewegung des Vorschub und Rückzug in longitudinaler Richtung wie auch die rechts oder links Drehung des Endoskops 2 in axialer Richtung in ein elektronisches Echt-Zeit Signal umwandelt.

Ein mehrteiliger Aufbau des DOME-Modules 3 zu Reinigungszwecken oder auch zum Austausch einzelner Komponenten ist möglich. Das DOME-Modul 3 kann vom Mundstück 12 getrennt werden, um es z.B. separat zu desinfizieren oder auch komplett auszutauschen.

In einem Mess-Signal Konverter, wie zum Beispiel ein Computer oder eine an einem Computer angeschlossene Recheneinheit, findet dann eine Umwandlung des digitalen Signales derart statt, dass in axialer Orientierung 8 eine auf einen Vollkreises von 360° begrenzte graphische Darstellung erfolgt, sowie der Vorschub des Endoskops 2 additiv und dahingegen ein Rückzug des Endoskops 2 als Subtraktion der longitudinalen Einführungstiefe 7 verarbeitet wird. Axialer und longitudinaler Messwert werden dann als real-time Messwerte zusammen mit dem Video-Signal des Endoskops 2 in einem Computer 17 zu einem Video-in-Video verarbeitet, welches dann als kombiniertes Video von Endoskopie 2 und der DOME-Messung auf einem Bildanzeigegerät 9 ausgegeben und synchron auf dem Computer 17 gespeichert werden kann.

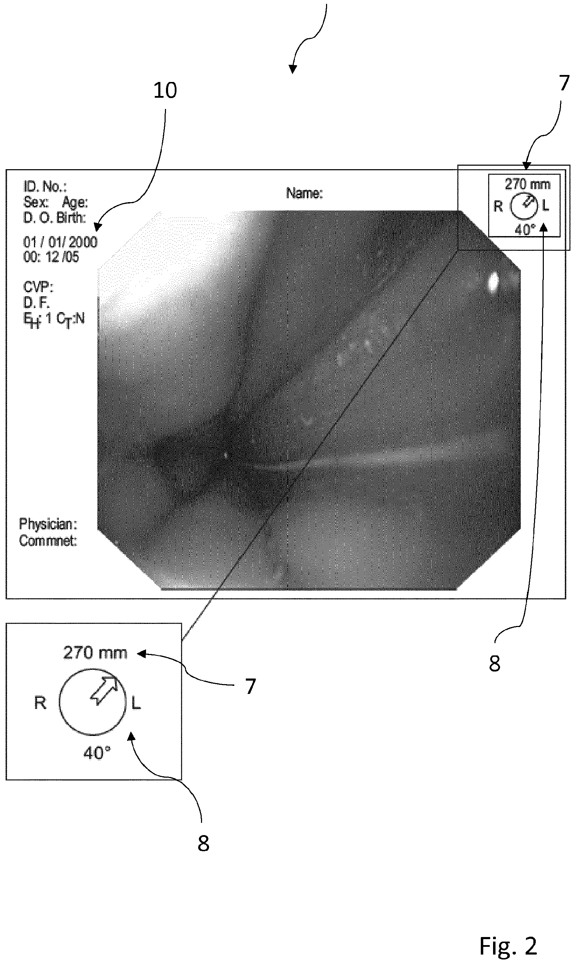

Figur 2 zeigt ein Bildanzeigegerät 9 während der Messung einer endoskopischen Untersuchung. Es wird deutlich, dass während der Untersuchung mit einem Endoskop 2 die longitudinale Einführungstiefe 7 und die axiale Orientierung 8 des Endoskops 2 auf dem Bildanzeigegerät 9 dargestellt werden kann. Diese Darstellung ist mit einem Zeitbezug 10 gekoppelt, der eine exakte Reproduktion der Daten ermöglicht.

Entscheidend bei allen Methoden ist die Umwandlung der Bewegung des Endoskops 2 im Verhältnis zum Mundstück 12 oder Anal-Einsatzes, bzw. zum Dome-Modul 3, in digitale Echt-Zeit Messdaten und deren Projektion VIDEO-in-VIDEO in die Videoaufnahme der endoskopischen Untersuchung.

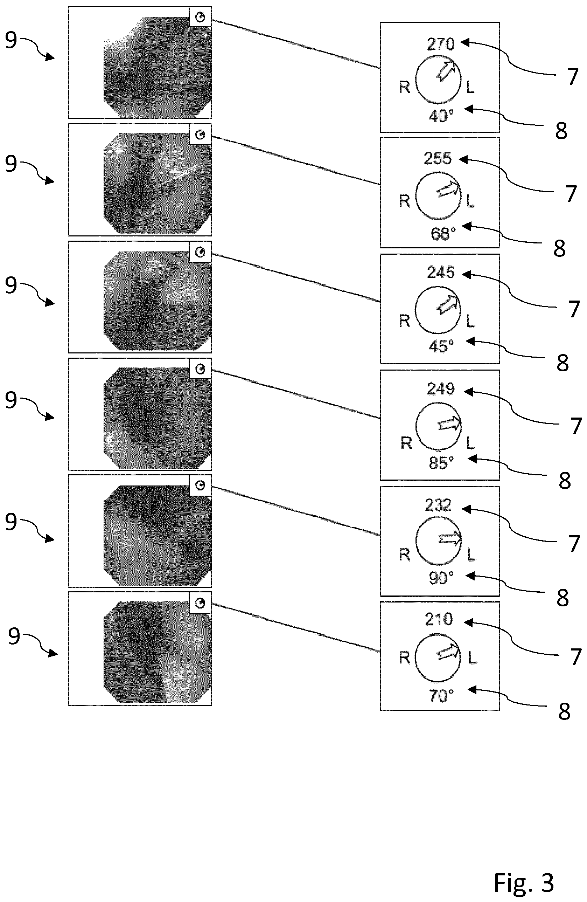

Figur 3 zeigt anhand von Einzelbildern eine beispielhafte Bilder-Sequenz einer DOME-Video-Endoskopie. Deutlich wird, dass zu jedem Zeitpunkt eine exakte Messung der Einführungstiefe 7 und axialen Orientierung 8 des Endoskops 2 erfolgen kann. Die so gewonnenen Daten können dabei auf einem Bildanzeigegerät 9 dargestellt oder zur späteren Verwendung auf einem Datenträger gespeichert werden.

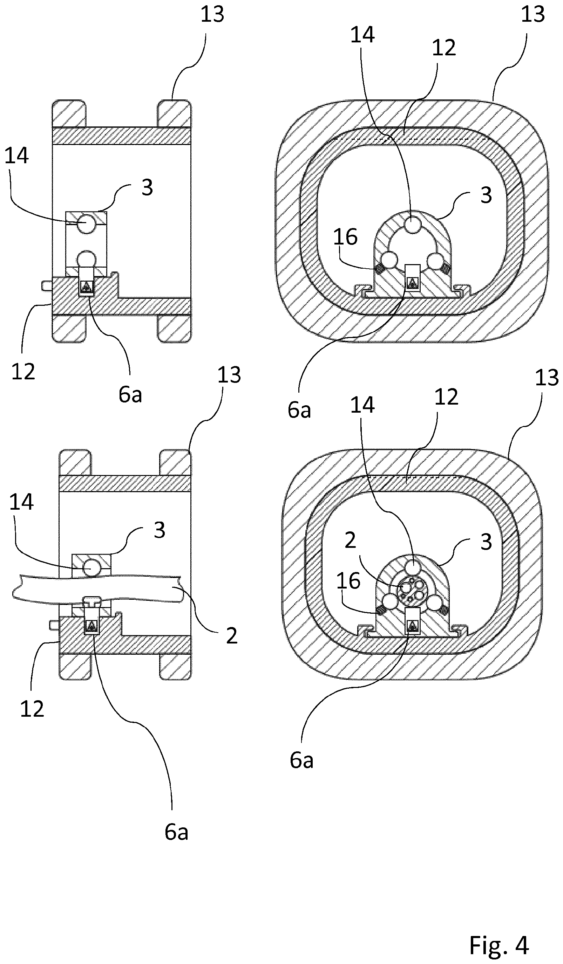

Figur 4 zeigt einen konzeptionellen Aufbau des Mundstückes 12 mit DOME-Modul 3 und einem Laser-Sensor 6a. Auf der linken Seite ist das Mundstück 12 in einer Seitenansicht und auf der rechten Seite ist das Mundstück 12 in einer Vorderansicht gezeigt. Oben ist das Dome-Modul 3 ohne eingeführtes Endoskop 2 und unten mit eingeführtem Endoskop 2 zu erkennen. Die Figur 4 zeigt somit dasselbe Mundstück 12 in vier unterschiedlichen Darstellungen.

Das DOME-Modul 3 wird dabei via Einschub auf einem Schlitten auf der Innenseite des Mundstückes 12 eingeschoben und gegen Verschub arretiert. Das DOME-Modul 3a kann z.B. aus Polyamid oder Polycarbonat oder einem anderen Polymer-Kunststoff gefertigt sein. Dabei ist im inneren Durchmesser des DOME-Modules 3 ein Kugellager 14 ausgebildet, wobei mindestens drei Kugeln vorhanden sind. Diese Kugeln haben direkten mechanischen Kontakt zum Endoskop 2 und halten das Endoskop 2 ohne den Vor- und Rückschub aber auch ohne die Rotation des Endoskops 2 im DOME-Moduls 3a zu beeinträchtigen auf konstantem Abstand zum Laser-Sensor 6a.

Hierfür sind mindestens zwei der Kugeln des Kugellagers 14 im DOME-Modul 3 mit einem Andruckmechanismus 16 versehen, z.B. Andruckfedern, sodass ein definiertes Spiel des Endoskops 2 im inneren Durchmesser des DOME-Modules 3 möglich ist, aber die Andruckkraft z.B. der Andruckfedern den Kontakt zu den Kugeln konstant aufrecht erhält. Der Laser-Sensor 6a registriert und misst jede Bewegung des Endoskops 2 im Verhältnis zum DOME-Modul 3 bzw. zum Mundstück 12 und wandelt die longitudinale Einführungstiefe 7 wie auch die axiale Orientierung 8 in ein digitales Signal um, welches dann via Kabel, Bluetooth oder auch WLAN an einen Computer weitergeleitet wird. Denkbar ist auch, dass die optische Messung mittels Laser Oberflächen-Abtastung (ähnliche einer optischen Computer-Maus) ausgeführt wird.

Es ist nicht zwingend erforderlich, dass der Sensor als Laser-Sensor 6a ausgebildet ist. Denkbar ist auch, dass der Sensor als Licht-Sensor oder sonstiger optischer Sensor ausgebildet ist. Dabei kann das Endoskop 2 von einer Lichtquelle, wie zum Beispiel ein Laser oder einer LED, angestrahlt werden, wobei das vom Endoskop 2 reflektierte Licht über den Sensor erfasst wird. Über die Messvorrichtung 5 werden die so erfassten Bilder verglichen und damit die Bewegungsdaten des Endoskops 2 ermittelt.

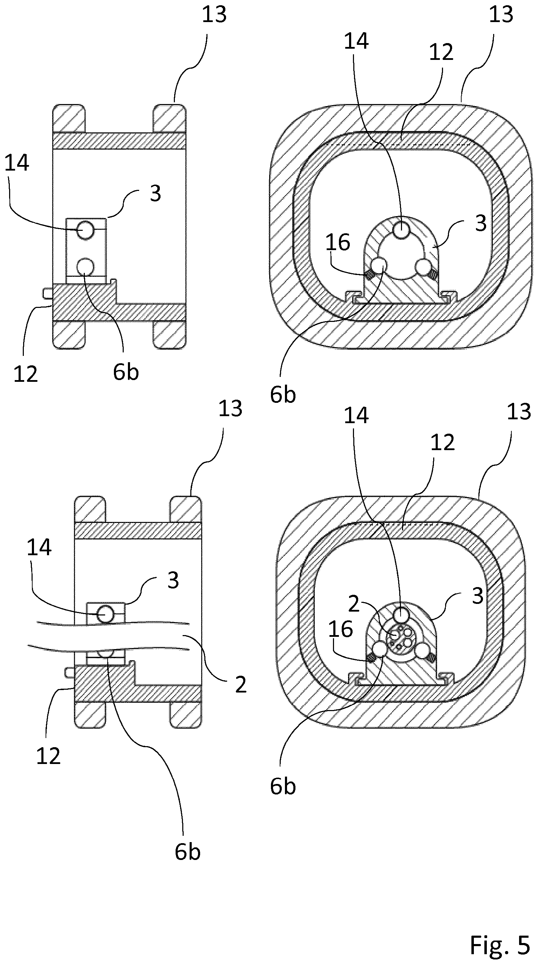

Figur 5 zeigt einen konzeptionellen Aufbau des Mundstückes 12 mit DOME-Modul 3 und einem Kugel-Sensor 6b. Auf der linken Seite ist das Mundstück 12 in einer Seitenansicht und auf der rechten Seite ist das Mundstück 12 in einer Vorderansicht gezeigt. Oben ist das Dome-Modul 3 ohne eingeführtes Endoskop 2 und unten mit eingeführtem Endoskop 2 zu erkennen. Die Figur 5 zeigt somit dasselbe Mundstück 2 in vier unterschiedlichen Darstellungen.

Die Messvorrichtung 5, bzw. der Kugel-Sensor 6b, am Mundstück 12 kann entweder direkten mechanischen Kontakt mit dem Endoskop 2 eingehen, z.B. im Sinne eines Kugel-Roll-Sensors 6b ähnlich dem einer analogen Computer-Maus. Dabei werden durch die Kugeln wenigstens zwei Wellen bewegt, die jeweils eine Scheibe mit Schlitzen am Ende der Welle in Rotation versetzen. Eine Lichteinheit und eine Fotozelle sind vor bzw. hinter den Schlitzen angeordnet. Durch die Wellen wird an den Schlitzen ein Takt aufgebaut, der grafisch umgesetzt werden kann.

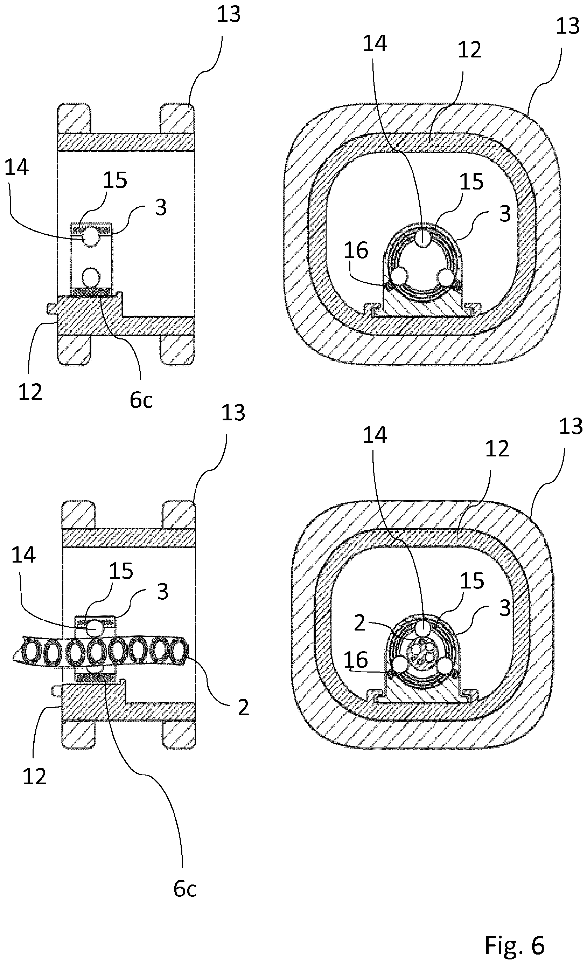

Figur 6 zeigt einen konzeptionellen Aufbau des Mundstückes 12 mit DOME-Modul 3 und einem Induktions-Sensor 6c, wobei durch eine Induktionsspule 15 am Mundstück 12 eine Spannung induziert wird, indem das mit Magnetring-Armierungen versehene Endoskop 2 durch die Induktionsspule 15 in den Körper des Patienten geführt wird. Über die induzierte Spannung kann dann auf die Einführungstiefe 7 geschlossen werden.

Auf der linken Seite ist das Mundstück 12 in einer Seitenansicht und auf der rechten Seite ist das Mundstück 12 in einer Vorderansicht gezeigt. Oben ist das Dome-Modul 3 ohne eingeführtes Endoskop 2 und unten mit eingeführtem Endoskop 2 zu erkennen. Die Figur 6 zeigt somit dasselbe Mundstück 2 in vier unterschiedlichen Darstellungen.

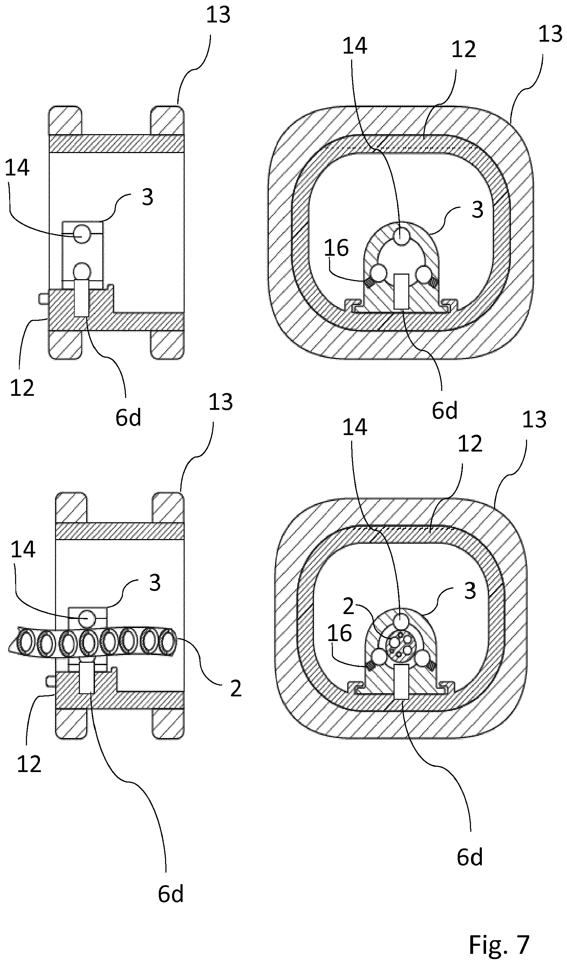

Figur 7 zeigt einen möglichen konzeptionellen Aufbau des Mundstückes 12 mit DOME-Modul 3 und einem Hall-Sensor 6d. Auf der linken Seite ist das Mundstück 12 in einer Seitenansicht und auf der rechten Seite ist das Mundstück 12 in einer Vorderansicht gezeigt. Oben ist das Dome-Modul 3 ohne eingeführtes Endoskop 2 und unten mit eingeführtem Endoskop 2 zu erkennen. Die Figur 7 zeigt somit dasselbe Mundstück 2 in vier unterschiedlichen Darstellungen.

Für die Auslegung mit Hall-Sensor 6d könnte das Endoskop 2 ringförmige magnetische Armierungen gleichbleibender oder auch unterschiedlicher aber fest definierter magnetischer Induktion aufweisen. Die Messung der longitudinalen Einführungstiefe 7 von Vorschub und Rückzug des Endoskops 2 könnte auch mittels optischem Sensor 6 und einer wiederholten ringförmigen schwarz-weißen Kontraststreifen-Armierung des Endoskops 2 ausgeführt werden.

Die Kombination von z.B. Kugel-Sensor 6b mit z.B. Hall-Sensor 6d oder Induktions-Sensor 6c ist ebenfalls möglich und ggf. zur Vereinfachung auch sinnvoll, da z.B. eine Messung der Bewegung in X- und Y-Achse (longitudinal und axial Bewegungen) einer besonderen elektronischen Signalauswertung notwendig machen kann.

Das Endoskop 2 muss für jede Ausführung des Sensiermittels 6 und den technischen Ausführungen des DOME-Moduls 3 gegebenenfalls speziell ausgelegt sein. Dies betrifft z.B. entweder ausreichende Oberflächenreibung für die Kugel-Sensor-Auslegung, eine ausreichend lichtstreuende Oberfläche im Falle einer Auslegung als Laser-Sensor 6a, eine gleichbleibende Magnetfeldstärke des Endoskops 2 über seine Länge oder Induktionsverstärkende Armierungen, die sich in fest definierten regelmäßigen Abständen (z.B. 0,5 cm) wiederholen im Falle eines Induktions-Sensors 6c.

Die Figuren verstehen sich als mögliche Ausführungsbeispiele. Andere Formen der erfindungsgemäßen Lehre sind weiterhin denkbar. Des Weiteren sind die Ausgestaltungen des Ausführungsbeispiels nicht untrennbar miteinander verknüpft, sodass z.B. die Ausführung der Erfindung nicht abhängig von den speziell beschriebenen Ausgestaltungen des Ausführungsbeispiels ist. So ist eine Variabilität zum Beispiel von der Art, Anzahl, Länge oder Größe der einzelnen Elemente jederzeit denkbar.

## Patentansprüche

1. Medizinische Vorrichtung (1), z.B. für die Endoskopie und/oder Intubation, mit einem länglichen, in eine Körperöffnung (4) eines Patienten einführbaren Hauptelement (2), wobei die medizinische Vorrichtung (1) ferner ein Referenzbauteil (3) aufweist, das dazu eingerichtet ist, vor dem Einführen des Hauptelements (2) in die Körperöffnung (4) an einer definierten Stelle nahe der Körperöffnung (4) am Patienten fixiert zu werden, wobei die medizinische Vorrichtung (1) ferner eine Messvorrichtung (5) aufweist, die über wenigstens ein Sensiermittel (6) die Einführungstiefe (7) des Hauptelements (2) und/oder die axiale Orientierung (8) des Hauptelements (2) relativ zum Referenzbauteil (3) erfasst und als Sensordaten zur weiteren Verarbeitung und/oder Speicherung bereitstellt.

2. Vorrichtung (1) nach Anspruch 1, **dadurch gekennzeichnet, dass** die Vorrichtung (1) ein Bildanzeigegerät (9) aufweist oder damit verbunden ist, wobei die Vorrichtung (1) dazu eingerichtet ist, in auf dem Bildanzeigegerät (9) dargestellten Bilddaten in Echtzeit die von der Messvorrichtung (5) bereitgestellten Sensordaten, die die Einführungstiefe (7) und/oder die axiale Orientierung (8) wiedergeben, einzublenden.

3. Vorrichtung (1) nach einem der vorhergehenden Ansprüche, **dadurch gekennzeichnet, dass** die Vorrichtung (1) dazu eingerichtet ist, die Sensordaten mit einem Zeitbezug (10) zu speichern, insbesondere mit einem Zeitbezug (10) zu den auf dem Bildanzeigegerät (9) dargestellten Bilddaten.

4. Vorrichtung (1) nach einem der vorhergehenden Ansprüche, **dadurch gekennzeichnet, dass** das längliche Hauptelement (2) an einem distalen Ende wenigstens eine Bilderfassungseinheit, insbesondere eine Kamera, hat.

5. Vorrichtung (1) nach Anspruch 4, **dadurch gekennzeichnet, dass** die Bilderfassungseinheit dazu eingerichtet ist, die Bilddaten an die Messvorrichtung (5) zur Verarbeitung der Bilddaten zu übertragen.

6. Vorrichtung (1) nach einem der vorhergehenden Ansprüche, **dadurch gekennzeichnet, dass** das längliche Hauptelement (2) durch wenigstens eine Ausnehmung (11) des Referenzbauteils (3) führbar ist.

7. Vorrichtung (1) nach Anspruch 6, **dadurch gekennzeichnet, dass** eine Null-Kalibrierung zur Bestimmung der Ausgangsposition des länglichen Hauptelementes (2) nach Einführung des länglichen Hauptelementes (2) in das Referenzbauteil (3) erfolgt.

8. Vorrichtung (1) nach einem der vorhergehenden Ansprüche, **dadurch gekennzeichnet, dass** das Referenzbauteil (3) ein Mundstück (12) hat, wobei das Mundstück (12) wenigstens einen Beißring (13) hat und dazu eingerichtet ist, das Referenzbauteil (3) zu fixieren.

9. Vorrichtung (1) nach einem der vorhergehenden Ansprüche, **dadurch gekennzeichnet, dass** das Referenzbauteil (3) zur verbesserten Reinigung mehrteilig aufgebaut ist.

10. Vorrichtung (1) nach einem der vorhergehenden Ansprüche, **dadurch gekennzeichnet, dass** das Sensiermittel (6) ein Laser-Sensor (6a) und/oder ein Kugel-Sensor (6b) und/oder eine Hall-Sonde (6d) und/oder ein Induktions-Sensor (6c) und/oder ein Licht-Sensor und/oder ein Optischer-Sensor und/oder ein Wälzrollen-Sensor ist.

11. Vorrichtung (1) nach einem der vorhergehenden Ansprüche, **dadurch gekennzeichnet, dass** das längliche Hauptelement (2) ein Katheter, ein Schlauch oder ein Endoskop (2) ist.
